(19) Europäisches Patentamt
European Patent Office
Office européen des brevets

(11) **EP 1 761 762 B1**

(12) **EUROPÄISCHE PATENTSCHRIFT**

(45) Veröffentlichungstag und Bekanntmachung des Hinweises auf die Patenterteilung:
**04.01.2012 Patentblatt 2012/01**

(21) Anmeldenummer: 05769280.8

(22) Anmeldetag: **30.06.2005**

(51) Int Cl.:
*G01N 27/403* (2006.01)     *C12Q 1/68* (2006.01)
*G01N 33/543* (2006.01)

(86) Internationale Anmeldenummer:
**PCT/DE2005/001163**

(87) Internationale Veröffentlichungsnummer:
**WO 2006/002617 (12.01.2006 Gazette 2006/02)**

(54) **PLANAR-SENSOR-ANORDNUNG, SENSOR-ARRAY UND VERFAHREN ZUM HERSTELLEN EINER PLANAR-SENSOR-ANORDNUNG**

PLANAR-SENSOR ARRANGEMENT, SENSOR ARRAY AND METHOD FOR THE PRODUCTION OF A PLANAR-SENSOR ARRANGEMENT

ENSEMBLE DETECTEUR PLAN, RESEAU DE DETECTEURS ET PROCEDE POUR PRODUIRE UN ENSEMBLE DETECTEUR PLAN

(84) Benannte Vertragsstaaten:
**DE FR GB**

(30) Priorität: **30.06.2004 DE 102004031672**

(43) Veröffentlichungstag der Anmeldung:
**14.03.2007 Patentblatt 2007/11**

(73) Patentinhaber: **SIEMENS AKTIENGESELLSCHAFT
80333 München (DE)**

(72) Erfinder:
• **FREY, Alexander
  81737 Muenchen (DE)**
• **PAULUS, Christian
  82362 Weilheim (DE)**
• **SCHIENLE, Meinrad
  85521 Ottobrunn (DE)**
• **THEWES, Roland
  82194 Gröbenzell (DE)**

(56) Entgegenhaltungen:
WO-A-03/083134      WO-A-2004/019024
WO-A2-01/75151      WO-A2-03/102602
DE-B- 1 029 820     US-A1- 2004 045 839
US-B1- 6 281 689    US-B1- 6 383 858

EP 1 761 762 B1

**Beschreibung**

Die Erfindung betrifft eine Planar-Sensor-Anordnung, ein Sensor-Array und ein Verfahren zum Herstellen einer Planar-Sensor-Anordnung.

Aus dem Stand der Technik sind Zweipol-Impedanz-Sensören für die Biosensorik bekannt, siehe z.B. [1] bis [8]. Ferner wurden Zweipol-Impedanz-Verfahren auch für die Sensorik anderer chemischer Stoffe, z.B. im Rahmen der Gassensorik, vorgeschlagen.

[9] offenbart CMOS-Sensor-Interface-Arrays für die DNA-Detektion.

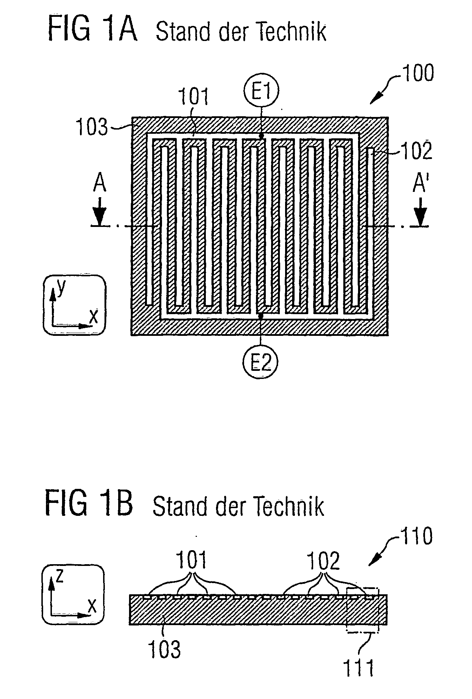

Im Weiteren wird bezugnehmend auf Fig.1A Fig.1B eine Sensor-Anordnung 100 gemäß dem Stand der Technik beschrieben.

In **Fig.1A** ist eine Draufsicht der Sensor-Anordnung 100 gezeigt, die in einem Silizium-Substrat 103 integriert ist. In einem ersten Oberflächenbereich des Silizium-Substrats 103 ist eine erste Interdigitalelektrode 101 vorgesehen. In einem anderen Oberflächenbereich des Silizium-Substrats 103 ist eine zweite Interdigitalelektrode 102 vorgesehen. Die Interdigitalelektroden 102, 103 sind fingerförmig ineinandergreifend ausgestaltet.

In **Fig.1B** ist eine Querschnittsansicht 110 der Sensor-Anordnung 100 entlang einer Schnittlinie A-A' gezeigt.

Die aus [4], [7], [8] bekannte Sensor-Anordnung 100 ist zum Durchführen einer Zweipolimpedanz-Messung im Rahmen der DNA-Sensorik verwendbar. Die Sensor-Elektrode 100 enthält zwei Interdigitalelektroden 101, 102 mit in einer Dimension periodisch nebeneinander angeordneten Fingern.

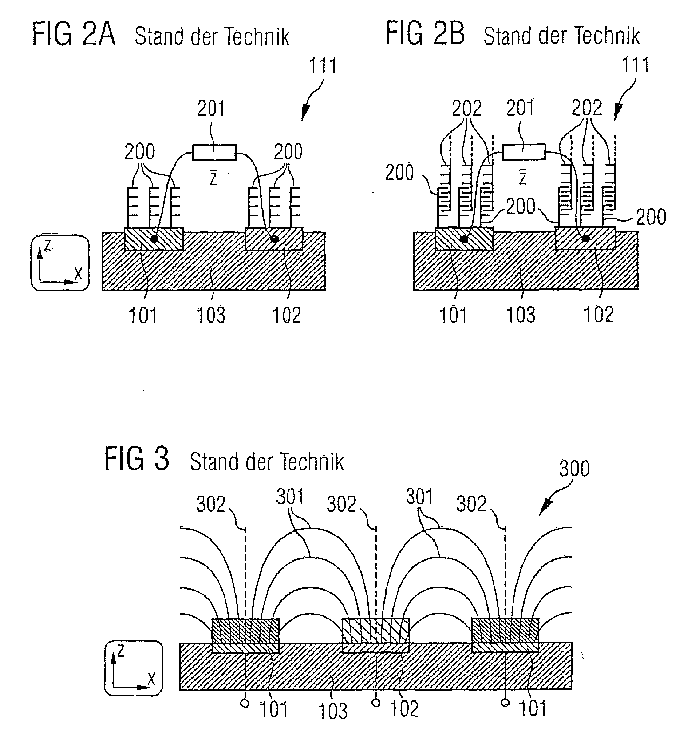

Ferner ist in Fig.1B ein Teilbereich 111 der Sensor-Anordnung 100 gezeigt, welcher zur Erläuterung des Prinzips und der Funktionsweise der Sensor-Anordnung 100 im Weiteren basierend auf Fig.2A, Fig.2B näher beschrieben wird.

In **Fig.2A** ist der Teilbereich 111 der Sensor-Anordnung 100 in einem ersten Betriebszustand gezeigt, in dem ein zu erfassende Partikel aufweisender Analyt mit der Sensor-Anordnung 100 nicht in Kontakt gebracht ist. An den Elektroden 101, 102 sind Fängermoleküle 200, z.B. DNA-Halbstränge immobilisiert. Als Material für die Interdigitalelektroden 101, 102 wird häufig Gold verwendet, so dass die Immobilisierung der Fängermoleküle 200 über die in der Biochemie vielfach verwendete Gold-Schwefel-Kopplung zwischen Goldmaterial der Interdigitalelektroden 101, 102 einerseits und Thiol-Gruppen (SH-Gruppen) der Fängermoleküle 200 andererseits erfolgt.

In **Fig.2B** ist ein zweiter Betriebszustand der Sensor-Anordnung 100 gezeigt, nachdem diese mit einem zu erfassende Partikel 202 aufweisenden Analyt in Kontakt gebracht worden ist. Oberhalb der Sensor-Elektroden 101, 102 befindet sich somit während des aktiven Sensorbetriebes der zu untersuchende Analyt, der aus einem Elektrolyten besteht, der möglicherweise zu erfassenden Partikel 202 aufweist. Eine Hybridisierung, wie sie in Fig.2B schematisch dargestellt ist, das heißt eine Anbindung von DNA-Halbsträngen 202 an die Fängermoleküle 200, findet nur dann statt, wenn Fängermoleküle 200 und DNA-Halbstränge 202 gemäß dem Schlüssel-Schloss-Prinzip zueinander passen, was als "Match" bezeichnet wird. Ist dieses nicht der Fall, so erfolgt keine Hybridisierung. Diese Situation wird als "Mismatch" bezeichnet. Die Spezifität des Sensors leitet sich somit aus der Spezifität der Fängermöleküle ab.

Der elektrische Parameter, der gemäß dem Sensor-Prinzip des Sensors gemäß Fig.2A, Fig.2B ausgewertet wird, ist die Impedanz 201 zwischen den Elektroden 101, 102, die in Fig.2A, Fig.2B schematisch gezeigt ist. Im Falle einer erfolgten Hybridisierung (gegebenenfalls nach einem auf die Hybridisierungsphase folgenden Spülschritt) ändert sich der Wert der Impedanz 201, da DNA-Moleküle 202 und Fängermoleküle 200 andere elektrische Eigenschaften aufweisen als der Elektrolyt, und da bei der Hybridisierung Elektrolyt-Material aus dem Bereich zwischen den Elektroden 101, 102 verdrängt wird.

Im Weiteren wird bezugnehmend auf **Fig.3** eine andere Teilansicht 300 der Sensor-Anordnung 100 aus Fig. 1B beschrieben, in der schematisch elektrische Feldlinien 301 zwischen den Elektroden 101, 102 eingezeichnet sind. Die elektrischen Feldlinienverläufe 301 der Interdigitalstrukturen 101, 102 weisen, wie in Fig.3 gezeigt, Symmetrielinien 302 auf, die für die analytische Beschreibung und Bewertung der Eigenschaften der Sensor-Anordnung verwendet werden können.

Allerdings weisen die aus dem Stand der Technik bekannten Sensor-Anordnungen auf Basis von Interdigitalelektroden, wie die in Fig.1A bis Fig.3 gezeigte Sensor-Anordnung, für manche Anwendungen keine ausreichende Nachweissensitivität auf.

In [10] wird ein Sensor zur qualitativen und quantitativen Bestimmung von (bio)organischen Oligomeren und Polymeren mit Detektionselektroden beschrieben, auf denen Fängermoleküle zum Hybridisieren mit zu bestimmenden organischen Oligomeren und Polymeren angeordnet sind, wobei die Detektionselektroden zwischen Attraktionselektroden angeordnet sind, welche den Analyt mit der zu erfassenden chemischen Verbindung mit Hilfe elektrischer Felder über die Sensoranordnung hinwegbewegen.

In [11] wird eine Sensor-Anordnung zur Basen-Sequenzierung gezeigt, in der Sensor-Gruppen spaltenweise abwechselnd angeordnet sind. Die einzelnen Sensor-Gruppen sind 3-Elektrodensysteme, welche jeweils eine Detektionselektrode bzw. eine Vergleichselektrode, eine Gegenelektrode und eine Referenzelektrode aufweisen.

In [12] wird eine Sensor-Anordnung mit Elektroden, auf denen Fängermoleküle immobilisiert sind, beschrieben.

In [13] werden eine Sensor-Anordnung und ein Verfahren zum Betreiben einer Sensor-Anordnung beschrieben.

Die Sensor-Anordnung weist mindestens drei Elektroden auf, wobei zumindest auf einem Teil der Elektroden Fängermoleküle immobilisiert sind.

In [14] wird eine IC-Anordnung mit einer Kondensatorstruktur beschrieben, welche aus einem Array von in zwei Dimensionen alternierenden ersten und zweiten Elektrodenelementen gebildet ist.

Der Erfindung liegt insbesondere das Problem zugrunde, eine Sensor-Anordnung mit einer gegenüber einer Interdigitalelektroden-Struktur erhöhten Nachweissensitivität bereitzustellen.

Dieses Problem wird durch eine Planar-Sensor-Anordnung, durch ein Sensor-Array und durch ein Verfahren zum Herstellen einer Planar-Sensor-Anordnung mit den Merkmalen gemäß den unabhängigen Patentansprüchen gelöst.

Die erfindungsgemäße Planar-Sensor-Anordnung zum Erfassen von in einem Analyten möglicherweise enthaltenen Partikeln enthält ein Substrat, eine erste Planar-Sensor-Elektrode und eine zweite Planar-Sensor-Elektrode, die auf und/oder in dem Substrat gebildet sind, und auf denen Fängermoleküle immobilisiert sind, wobei die erste Planar-Sensor-Elektrode und die zweite Planar-Sensor-Elektrode jeweils in eine Mehrzahl von planaren Sensor-Elektroden-Teilbereichen aufgeteilt sind, wobei die Sensor-Elektroden-Teilbereiche der ersten Sensor-Elektrode und die Sensor-Elektroden-Teilbereiche der zweiten Sensor-Elektrode in der Oberflächenebene des Substrats in zwei Dimensionen alternierend angeordnet sind. Ferner enthält die Planar-Sensor-Anordnung eine Verdrahtungsstruktur, mittels welcher zumindest ein Teil der Sensor-Elektroden-Teilbereiche der ersten Sensor-Elektrode miteinander elektrisch gekoppelt sind, und mittels welcher zumindest ein Teil der Sensor-Elektroden-Teilbereiche der zweiten Sensor-Elektrode miteinander elektrisch gekoppelt sind.

Ferner ist erfindungsgemäß ein Sensor-Array mit einer Mehrzahl von auf und/oder in dem Substrat gebildeten Planar-Sensor-Anordnungen mit den oben beschriebenen Merkmalen geschaffen.

Bei dem erfindungsgemäßen Verfahren zum Herstellen einer Planar-Sensor-Anordnung zum Erfassen von in einem Analyten möglicherweise enthaltenen Partikeln wird eine erste planare Sensor-Elektrode und eine zweite planare Sensor-Elektrode auf und/oder in einem Substrat gebildet, auf denen Fängermoleküle immobilisiert werden, wobei die erste Sensor-Elektrode und die zweite Sensor-Elektrode jeweils in eine Mehrzahl von planaren Sensor-Elektroden-Teilbereichen aufgeteilt werden, wobei die Sensor-Elektroden-Teilbereiche der zweiten Sensor-Elektrode in der Oberflächenebene des Substrats in zwei Dimensionen alternierend angeordnet werden. Ferner wird eine Verdrahtungsstruktur gebildet, mittels welcher zumindest ein Teil der Sensor-Elektroden-Teilbereiche der ersten Sensor-Elektroden miteinander elektrisch gekoppelt werden, und mittels welcher zumindest ein Teil der Sensor-Elektroden-Teilbereiche der zweiten Sensor-Elektrode miteinander elektrisch gekoppelt werden.

Eine Grundidee der Erfindung ist darin zu sehen, anstelle der aus dem Stand der Technik bekannten Interdigitalelektroden-Struktur zwei planare Sensor-Elektroden vorzusehen, die jeweils aus mehreren in einer Oberflächenebene des Substrats gebildeten und in dieser Ebene vorzugsweise nichtzusammenhängenden Sensor-Elektroden-Teilbereichen gebildet sind. Die Sensor-Elektroden-Teilbereiche der beiden Sensor-Elektroden sind in zwei Dimensionen, nämlich in zwei vorzugsweise orthogonalen Richtungen in der Oberflächenebene der Sensor-Anordnung, alternierend (z.B. schachbrettartig) angeordnet. Anders ausgedrückt wechseln sich entlang einer ersten Richtung in der Oberflächenebene des Substrats erste und zweite Sensor-Elektroden-Teilbereiche ab, und es wechseln sich entlang einer zweiten Richtung in der Oberflächenebene des Substrats (die zu der ersten Richtung vorzugsweise orthogonal verläuft) erste und zweite Sensor-Elektroden-Teilbereiche ab. In einem Umgebungsbereich eines Sensor-Elektroden-Teilbereichs der ersten Sensor-Elektrode ist somit mindestens ein Sensor-Elektroden-Teilbereich der zweiten Sensor-Elektrode gebildet. Wie sich mathematisch zeigen lässt (siehe Gleichungen (1) bis (42)), weist eine solche Planar-Sensor-Anordnung für viele Szenarien eine höhere Nachweissensitivität auf als die Interdigitalelektroden-Anordnung gemäß dem Stand der Technik.

Die vollkommen planare Oberflächenstruktur der Sensor-Anordnung der Erfindung ist prozesstechnisch viel einfacher fertigbar als eine dreidimensionale Elektrodenstruktur.

Bevorzugte Weiterbildungen der Erfindung ergeben sich aus den abhängigen Ansprüchen.

Die Sensor-Elektroden-Teilbereiche der Planar-Sensor-Anordnung können im Wesentlichen matrixförmig angeordnet sein. Gemäß dieser Ausgestaltung können die Sensor-Elektroden-Teilbereiche in Zeilen und in Spalten angeordnet sein, so dass sich anschaulich ein schachbrettartiges Muster aus ersten und zweiten Sensor-Elektroden-Teilbereichen ergibt. Von Sensor-Elektroden-Teilbereichen an Randabschnitten der Planar-Sensor-Elektroden abgesehen ist gemäß dieser Ausgestaltung ein Sensor-Elektroden-Teilbereich der ersten Sensor-Elektrode von vier Sensor-Elektroden-Teilbereichen der zweiten Sensor-Elektrode als nächste Nachbarn umgeben.

Zumindest ein Teil der Sensor-Elektroden-Teilbereiche in der Oberflächenebene des Substrats kann eine Ausdehnung aufweisen, die kleiner ist als der Abstand dieses Sensor-Elektroden-Teilbereichs zu mindestens einem unmittelbar benachbarten Sensor-Elektroden-Teilbereichs. Wie mathematisch gezeigt werden kann (siehe Gleichungen (1) bis (42)), kann bei der erfindungsgemäßen Planar-Sensor-Anordnung die Änderung der Impedanz infolge eines Sensorereignisses (z.B. einer Hybridisierung), insbesondere die Änderung des imaginären Anteils der Impedanz infolge eines Sensorereignisses, und somit die Nachweissensitivität der Planar-Sensor-Anordnung erhöht werden, wenn die Ausdehnung der Sensor-Elektroden-Teilbereiche kleiner gewählt wird als der Abstand unmittelbar benachbarter Sensor-Elektroden-Teilbereiche.

Auf der ersten planaren Sensor-Elektrode und/oder auf der zweiten planaren Sensor-Elektrode können Fängermoleküle immobilisiert sein. Solche Fängermoleküle können insbesondere DNA-Halbstränge, Proteine, Polypeptide oder sonstige Moleküle sein, die mit in einem zu untersuchten Analyten enthaltenen zu erfassenden Partikeln eine beliebige Bindung eingehen können (z.B. mit zu erfassenden Partikeln hybridisieren können).

Bei der Planar-Sensor-Anordnung der Erfindung kann zumindest ein Teil der Sensor-Elektroden-Teilbereiche in der Oberflächenebene des Substrats polygone Gestalt (d.h. die Gestalt eines Polygons) aufweisen.

Insbesondere kann zumindest ein Teil der Sensor-Elektroden-Teilbereiche in der Oberflächenebene des Substrats rechteckige oder quadratische Gestalt bzw. Form aufweisen.

Darüber hinaus kann zumindest ein Teil der Sensor-Elektroden-Teilbereiche der Planar-Sensor-Anordnung in der Oberflächenebene des Substrats dreieckige Gestalt aufweisen.

Gemäß der zuletzt beschriebenen Ausgestaltung kann zumindest ein Teil der Sensor-Elektroden-Teilbereiche in der Oberflächenebene des Substrats die Gestalt von gleichseitigen Dreiecken aufweisen, wobei die Dreieckseiten benachbarter Sensor-Elektroden-Teilbereiche zueinander parallel angeordnet sein können.

Alternativ oder ergänzend kann zumindest ein Teil der Sensor Elektroden-Teilbereiche in der Oberflächenebene des Substrats die Gestalt von gleichschenkligen Dreiecken aufweisen.

Alternativ oder ergänzend kann zumindest ein Teil der Sensor-Elektroden-Teilbereiche in der Oberflächenebene des Substrats die Gestalt von rechtwinkligen Dreiecken aufweisen, wobei die Dreieckseiten benachbarter Sensor-Elektroden-Teilbereiche zueinander parallel angeordnet sind.

Ferner kann zumindest ein Teil der Sensor-Elektroden-Teilbereiche in der Oberflächenebene des Substrats runde oder elliptische Gestalt oder Form aufweisen.

Die Verdrahtungsstruktur der Planar-Sensor-Anordnung der Erfindung kann als vergrabene elektrisch leitfähige Struktur im Inneren des Substrats gebildet sein.

In der Ebene, in der die Verdrahtungsstruktur als vergrabene elektrisch leitfähige Struktur gebildet ist, kann mindestens ein zusätzliches integriertes Bauelement (z.B. ein Feldeffekttransistor) gebildet sein. Somit kann eine elektrisch aktive Ebene mit der Verdrahtungsstruktur und zusätzlichen integrierten Bauelementen als vergrabene Struktur unterhalb der Sensor-Elektroden vorgesehen sein, was eine besonders platzsparende Ausgestaltung darstellt. Dadurch ist kostbare Substratfläche (z.B. Silizium-Substrat oder Silizium-Chip) gespart und eine miniaturisierte Sensor-Anordnung geschaffen.

Das mindestens eine zusätzliche integrierte Bauelement kann ein CMOS-Bauelement sein. Anders ausgedrückt kann die Planar-Sensor-Anordnung als CMOS-Planar-Sensor-Anordnung eingerichtet sein.

Die Sensor-Elektroden-Teilbereiche der ersten Sensor-Elektrode und der zweiten Sensor-Elektrode können alternierend und matrixförmig in Zeilen und Spalten angeordnet sein, und die Verdrahtungsstruktur kann entlang einer Verlaufsrichtung gebildet sein, die mit Verlaufsrichtungen der Zeilen und Spalten einen Winkel von im Wesentlichen 45° einschließt. Gemäß dieser Ausgestaltung ist eine besonders platzsparende und effektive Verdrahtung und elektrische Ansteuerung der Sensor-Elektroden-Teilbereiche ermöglicht.

Die Planar-Sensor-Anordnung kann als Biosensor-Anordnung eingerichtet sein, alternativ auch als Chemosensor-Anordnung.

Insbesondere kann die Planar-Sensor-Anordnung als Impedanz-Serisor-Anordnung eingerichtet sein, d.h. derart eingerichtet sein, dass eine Veränderung der Impedanz zwischen den Sensor-Elektroden infolge eines Hybridisierungsereignisses messtechnisch erfasst wird. Das Erfassen der Impedanz kann insbesondere das Erfassen des Imaginärteils der Impedanz beinhalten, so dass anschaulich eine Änderung der Kapazität zwischen den Sensor-Elektroden erfasst wird.

Die Planar-Sensor-Anordnung der Erfindung kann als monolithisch integrierte Sensor-Anordnung eingerichtet sein, d.h. insbesondere in einem Halbleiter-Substrat integriert sein. Als Substrat kann insbesondere ein Silizium-Wafer oder ein Silizium-Chip verwendet werden.

Ferner kann die Planar-Sensor-Anordnung der Erfindung eine Schaltkreis-Einrichtung aufweisen, mittels welcher die erste Sensor-Elektrode und die zweite Sensor-Elektrode ansteuerbar sind.

Mittels der Schaltkreis-Einrichtung kann an die Sensor-Elektroden ein zeitlich veränderliches elektrisches Ansteuersignal anlegbar sein und von den Sensor-Elektroden ein zeitlich veränderliches elektrisches Detektionssignal erfassbar sein.

Ferner kann mindestens ein auf und/oder in dem Substrat gebildetes Verstärker-Element und/oder mindestens ein auf und/oder in dem Substrat gebildetes Auswerte-Element in der Planar-Sensor-Anordnung enthalten sein. Diese elektrischen Bauelemente können insbesondere als integrierte elektrische Bauelemente vorgesehen sein und zum Verstärken, Auswerten und/oder Vorverarbeiten eines Sensor-Signals dienen.

Die Ausgestaltungen wurden bezugnehmend auf die Planar-Sensor-Anordnung der Erfindung beschrieben. Diese Ausgestaltungen gelten auch für das Planar-Sensor-Anordnungen aufweisende Sensor-Array. Ferner gelten die Ausgestaltungen der Planar-Sensor-Anordnung auch für das Verfahren zum Herstellen einer Planar-Sensor-Anordnung.

Ausführungsbeispiele der Erfindung sind in den Figuren dargestellt und werden im Weiteren näher erläutert.
Es zeigen:

| | |
|---|---|
| Figuren 1A und 1B | eine Draufsicht und eine Querschnittsansicht einer Sensor-Anordnung mit Interdigitalelektroden gemäß dem Stand der Technik, |
| Figuren 2A, 2B | zwei Betriebszustände eines Teilbereichs der in Figuren 1A, 1B gezeigten Sensor-Anordnung mit Interdigitalelektroden gemäß dem Stand der Technik, |
| Figur 3 | eine Querschnittsansicht eines anderen Teilbereichs der in Figuren 1A, 1B gezeigten Sensor-Anordnung mit Interdigitalelektroden gemäß dem Stand der Technik, |
| Figur 4 | einen vergrößerten Teilbereich einer Sensor-Anordnung mit Interdigitalelektroden gemäß dem Stand der Technik, |
| Figur 5 | einen anderen vergrößerten Teilbereich der Sensor-Anordnung mit Interdigitalelektroden gemäß dem Stand der Technik, |
| Figur 6 | ein schematisches Ersatzschaltbild eines Teilbereichs einer Sensor-Anordnung mit Interdigitalelektroden gemäß dem Stand der Technik, |
| Figur 7 | ein anderes schematischen Ersatzschaltbild einer Sensor-Anordnung mit Interdigitalelektroden gemäß dem Stand der Technik, |
| Figur 8 | eine Planar-Sensor-Anordnung gemäß einem ersten Ausführungsbeispiel der Erfindung, |
| Figur 9 | eine andere Ansicht der Planar-Sensor-Anordnung gemäß dem ersten Ausführungsbeispiel der Erfindung, |
| Figur 10 | einen vergrößerten Teilbereich der Planar-Sensor-Anordnung gemäß dem ersten Ausführungsbeispiel der Erfindung, |
| Figur 11 | einen anderen vergrößerten Teilbereich der Planar-Sensor-Anordnung gemäß dem ersten Ausführungsbeispiel der Erfindung, |
| Figuren 12A bis 12C | schematische Bilder mit dem elektrischen Feldlinienverlauf einer Interdigitalelektroden-Struktur gemäß dem Stand der Technik und einer Planar-Sensor-Anordnung gemäß der Erfindung, |
| Figur 13 | eine Planar-Sensor-Anordnung gemäß einem zweiten Ausführungsbeispiel der Erfindung, |
| Figur 14 | eine Planar-Sensor-Anordnung gemäß einem dritten Ausführungsbeispiel der Erfindung, |
| Figur 15 | eine Planar-Sensor-Anordnung gemäß einem vierten Ausführungsbeispiel der Erfindung. |

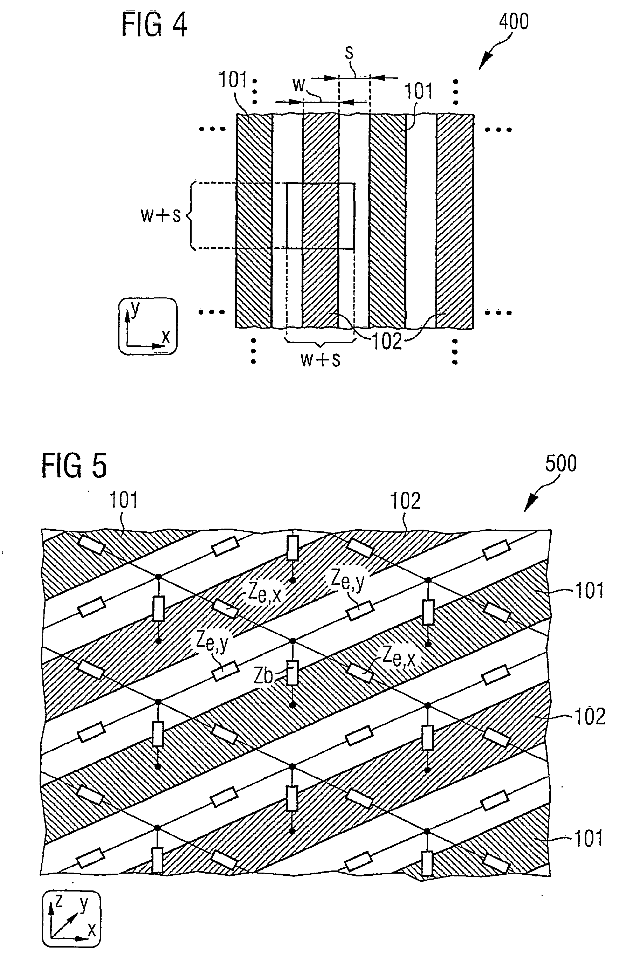

Gleiche oder ähnliche Komponenten in unterschiedlichen Figuren sind mit gleichen Bezugsziffern versehen.
Die Darstellungen in den Figuren sind schematisch und nicht maßstäblich.
Im Weiteren wird bezugnehmend auf Fig.4 bis Fig.7 eine mathematische Beschreibung des Sensorprinzips einer aus dem Stand der Technik bekannten Interdigitalelektroden-Struktur beschrieben. Bei dieser mathematischen Beschreibung wird die relative Änderung der Impedanz einer Interdigital-Elektroden-Sensor-Anordnung gemäß dem Stand der Technik (wie der Sensor-Anordnung 100) bei Detektion eines Hybridisierungsereignisses betrachtet.
In **Fig.4** ist nochmals ein Teilbereich 400 der Sensor-Anordnung 100 aus Fig.1 gezeigt, bei dem die gemäß Fig.4 in horizontaler Richtung abwechselnd angeordneten Elektrodenfinger der ersten Interdigitalelektrode 101 und der zweiten Interdigitalelektrode 102 gezeigt sind. Die Breite (oder Weite) einer Elektrode wird mit w bezeichnet, der Abstand zwischen benachbarten Elektroden wird mit s bezeichnet. Ferner wird ein Flächenausschnitt der Größe (w+s)x(w+s) betrachtet, welcher mittig über einer Sensor-Elektrode 101, 102 angeordnet ist. Aus herstellungstechnischen Gründen (v.a. bedingt durch Rahmenbedingungen der Lithographie) werden häufig für s und für w ähnliche Werte verwendet.
In **Fig.5** ist nochmals eine Draufsicht 500 der Sensor-Anordnung 100 gezeigt. Fig.5 stellt ein Ersatzschaltbild mit konzentrierten Elementen dar. In Fig.5 gezeigt sind eine Impedanz $Z_b$, deren Wert sich infolge eines Hybridisierungsereignisses ändert, und es sind die Elektrolyt-Impedanzen $Z_{e,x}$ und $Z_{e,y}$ in x-Richtung und in y-Richtung gezeigt. Da die Potentialverteilung oberhalb der Sensor-Anordnung 100, d.h. in dem Elektrolyten, aus Symmetriegründen zumindest in guter Näherung keine y-Abhängigkeit aufweist, können die Impedanzen $Z_{e,y}$ bei einer weiteren Vereinfachung des Ersatzschaltbildes vernachlässigt werden.

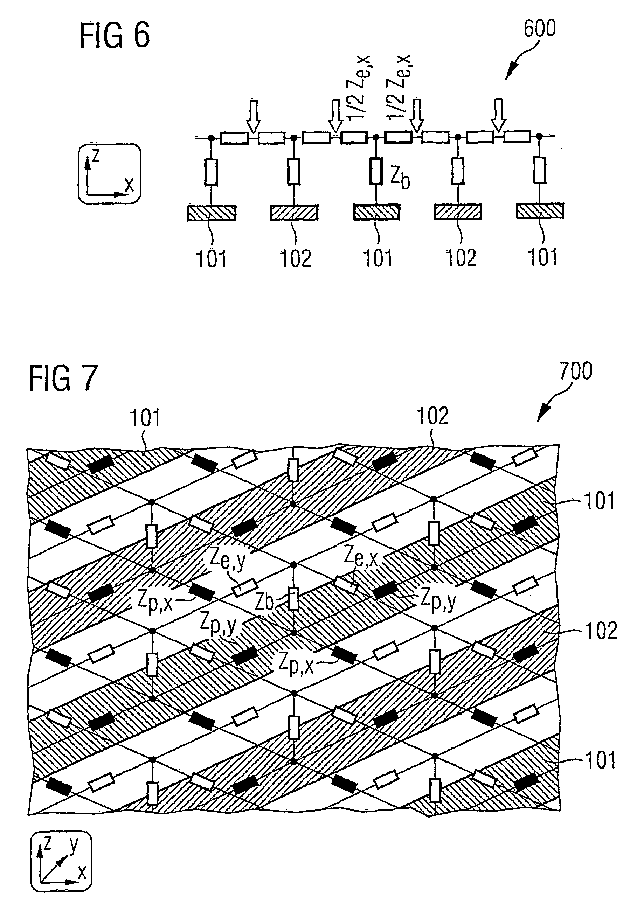

Dadurch ergibt sich die in dem Ersatzschaltbild 600 in **Fig.6** gezeigte Konfiguration. Es ist anzumerken, dass alle mit dem Symbol "Z" bezeichneten Parameter in der allgemeinen Darstellung komplexe Größen sind, d.h. Größen mit einem Realteil und mit einem Imaginärteil. Die in Fig.6 mit $Z_b$ bzw. $1/2Z_{e,x}$ bezeichneten Komponenten werden mit einer Sensor-Elektrode (in Fig.6 mit einem zugeordneten Finger der ersten Interdigitalelektrode 101) assoziiert. Der Vorfaktor 1/2 bei den Elektrolyt-Widerständen resultiert daraus, dass diese Widerstandskomponente jeweils zur Hälfte beiden Elektroden 101, 102 zugeordnet wird, oberhalb welcher sie sich in Fig.5 befindet.

Da die Werte der Impedanz $Z_b$ und $1/2Z_{e,x}$ von der Wahl der Parameter w und s abhängen, wird als Normgröße die Impedanz $Z_{b,0}$ eingeführt, die den (hybridisierungsabhängigen) Wert der Impedanz einer Elektrode 101, 102 der Fläche (w+s)x(w+s) hat, und die Square-Impedanz des Elektrolyten $Z_{e,sqr}$. Somit ergibt sich

$$Z_b = Z_{b,0} \times [ (w+s)^2 / ( (w+s) \times w) ] \qquad (1)$$

$$1/2 Z_{e,x} = Z_{e,sqr} \times [ 1/2 (w+s) / (w+s) ] \qquad (2)$$

und somit für den mit einer Elektrode 101, 102 assoziierten Wert der Impedanz $Z_{sens}$ :

$$Z_{sens} = Z_{b,0} \times [ (w+s)^2 / ( (w+s) \times w) ] + 1/2 Z_{e,sqr} \times [ 1/2 (w+s) / (w+s) ] \qquad (3)$$

$$Z_{sens} = Z_{b,0} \times [ 1 + s/w ] + 1/4 Z_{e,sqr} \qquad (4)$$

Der Vorfaktor 1/2 vor dem zweiten Term in Gleichung (3) ergibt sich aufgrund der Tatsache, dass an allen mit einem Pfeil gekennzeichneten Knoten (siehe Fig.6) aus Symmetriegründen in guter Näherung das gleiche elektrische Potential anliegt und diese Knoten somit als gemeinsamer Knoten betrachtet werden können. Dies entspricht einer Parallelschaltung der verbleibenden Elektrolyt-Widerstände gemäß Gleichung (2).

Die durchgeführte Näherung ist mathematisch und physikalisch sinnvoll, insbesondere wenn das Verhältnis s/w nicht allzu kleine Werte annimmt. Diese Anforderung an die Gültigkeit der Näherung ist jedoch durch die bereits zuvor besprochenen technologischen Rahmenbedingungen (insbesondere aus Gründen der Lithographie werden s und w häufig ähnlich gewählt) auch in vielen Szenarien erfüllt.

Um die Impedanz zu messen, wird an eine (oder an beide) der Elektroden 101, 102 eine (z.B. gegenphasige) Wechselspannung angelegt, und es wird das an den Elektroden resultierende Wechselstromsignal ausgewertet. Im Idealfall besitzt die komplexe Impedanz $Z_{b,0}$ nur kapazitive Komponenten. Sofern sie ohmsche Komponenten aufweist, sind diese als Parasitäreffekte anzusehen. In diesem Fall wird die Betriebsfrequenz der Sensor-Anordnung mittels Wählens einer hinreichend hohen Frequenz in einen Bereich gebracht, in dem die Impedanz $Z_{b,0}$ durch die kapazitiven Komponenten dominiert wird. Der Wert der assoziierten Kapazität wird als $C_{b,0}$ bezeichnet.

Die Impedanz des Elektrolyten setzt sich aus ohmschen Komponenten und kapazitiven Komponenten zusammen. Bei mittleren Frequenzen dominiert der ohmsche Anteil, der im Folgenden über dem Parameter $R_{e,sqr}$ modelliert wird, bei hohen Frequenzen dominiert der kapazitive Armteil, im Folgenden über $C_{e,sqr}$ modelliert.

Mit Hilfe dieser Größen kann für mittlere Frequenzen ($\omega = 2\pi f$) für den mit einer Elektrode assoziierten Wert der Impedanz $Z_{sens}$ der Näherungswert $Z_{sens}'$ angegeben werden:

$$Z_{sens}' = 1/j\omega C_{b,0} \times [ 1 + s/w ] + 1/4 R_{e,sqr} \qquad (5)$$

Für hohe Frequenzen kann der Wert der Impedanz $Z_{sens}$ angenähert werden durch $Z_{sens}''$ :

$$Z_{sens}''=1/j\omega\times[C_{b,0}\times(1+s/w)+1/4\times1/C_{e,sqr}] \tag{6}$$

Je nachdem, welche elektrische bzw. signalverarbeitende Auswertungsmethode für den Messwert von $Z_{sens}$ verwendet wird, kann der Betrag $|Z_{sens}|$ oder bei phasensensitiver Auswertung der Imaginärteil $Im(Z_{sens})$ betrachtet werden. Mit Gleichungen (5), (6) ergibt sich für mittlere Frequenzen:

$$|Z_{sens}'| = \sqrt{\left(\frac{1+s/w}{\omega C_{b,0}}\right)^2 + \left(\frac{1}{4}\times R_{e,sqr}\right)^2} \tag{7}$$

$$Im(Z_{sens}')=1/\omega C_{b,0}\times[1+s/w] \tag{8}$$

Für hohe Frequenzen sind Betrag und Imaginärteil gleich, d.h. es wird erhalten:

$$|Z_{sens}''|=1/\omega\times[1/C_{b,0}\times(1+s/w)+1/4\times1/C_{e,sqr}] \tag{9}$$

$$Im(Z_{sens}'')=1/\omega\times[C_{b,0}\times(1+s/w)+1/4\times1/C_{e,sqr}] \tag{10}$$

Um die Sensitivität des Sensors mit Interdigitalelektroden gemäß dem Stand der Technik zu bewerten, wird die Ableitung des Betrags bzw. des Imaginärteils der Impedanz nach dem Betrag von $Z_{b,0}$ normiert auf den Betrag bzw. den Imaginärteil der Impedanz betrachtet, die als relative Empfindlichkeit $\eta_{abs}$ bzw. $\eta_{im}$ definiert wird:

$$\eta_{abs}=\frac{\partial|Z_{sens}|/\partial|Z_{b,0}|}{|Z_{sens}|} \tag{11}$$

bzw.

$$\eta_{im}=\frac{\partial Im(Z_{sens})/\partial|Z_{b,0}|}{Im(Z_{sens})} \tag{12}$$

Für die Sensor-Anordnung mit Interdigitalelektroden gemäß Fig.1A bis Fig.5 ergibt sich für mittlere Frequenzen

$$\eta_{abs,1}'=\frac{\partial|Z_{sens}|/\partial|Z_{b,0}|}{|Z_{sens}|}=\frac{\partial\left(\sqrt{\left(\frac{1+s/w}{\omega C_{b,0}}\right)^2+\left(\frac{1}{4}\times R_{e,sqr}\right)^2}\right)\Big/\partial\left(\frac{1}{\omega C_{b,0}}\right)}{\sqrt{\left(\frac{1+s/w}{\omega C_{b,0}}\right)^2+\left(\frac{1}{4}\times R_{e,sqr}\right)^2}} \tag{13}$$

$$\eta_{abs,1}' = \frac{\omega C_{b,0}}{1+\left(\frac{1}{4}\times\frac{1}{1+s/w}\times\omega C_{b,0} R_{e,sqr}\right)^2} \quad (14)$$

und

$$\eta_{im,1}' = \frac{\frac{\partial Im(Z_{sens})}{\partial |Z_{b,0}|}}{Im(Z_{sens})} = \frac{\partial\left(\frac{1+s/w}{\omega C_{b,0}}\right)\Big/\partial\left(\frac{1}{\omega C_{b,0}}\right)}{\left(\frac{1+s/w}{\omega C_{b,0}}\right)} \quad (15)$$

$$\eta_{im,1}' = \omega C_{b,0} \quad (16)$$

Für hohe Frequenzen wird erhalten:

$$\eta_{abs,1}'' = \frac{\partial|Z_{sens}|\Big/\partial|Z_{b,0}|}{|Z_{sens}|} = \frac{\partial\left[\frac{1}{\omega}\times\left(1/C_{b,0}\times\left(1+\frac{s}{w}\right)+\frac{1}{4}\times 1/C_{e,sqr}\right)\right]\Big/\partial\left(\frac{1}{\omega C_{b,0}}\right)}{\frac{1}{\omega}\times\left(1/C_{b,0}\times\left(1+\frac{s}{w}\right)+\frac{1}{4}\times 1/C_{e,sqr}\right)} \quad (17)$$

$$\eta_{abs,1}'' = \frac{\omega C_{b,0}}{1+\frac{1}{4}\times\frac{1}{1+s/w}\times\frac{C_{b,0}}{C_{e,sqr}}} \quad (18)$$

und aufgrund der Identität von Gleichungen (9) und (10):

$$\eta_{im,1}'' = \frac{\frac{\partial Im(Z_{sens})}{\partial|Z_{b,0}|}}{Im(Z_{sens})} = \frac{\omega C_{b,0}}{1+\frac{1}{4}\times\frac{1}{1+s/w}\times\frac{C_{b,0}}{C_{e,sqr}}} \quad (19)$$

In **Fig.7** ist eine andere Draufsicht 700 der Sensor-Anordnung 100 gezeigt, die ein gegenüber Fig.5 erweitertes Ersatzschaltbild zeigt. Zusätzlich zu der Impedanz $Z_b$, deren Wert sich infolge eines Hybridisierungsereignisses ändert, und den Elektrolytimpedanzen $Z_{e,x}$ und $Z_{e,y}$ in x- und y-Richtung sind in Fig.7 noch die Parasitärelemente $Z_{p,x}$ und $Z_{p,y}$ in x-und y-Richtung dargestellt, die für die Impedanzen stehen, die sich direkt in der Ebene der Elektroden 101, 102 und des Trägermaterials 103 der Sensor-Elektroden 101, 102 ergeben. Diese Komponenten können rechnerisch ähnlich berücksichtigt werden, wie dies mit Hilfe der Gleichungen (1) bis (19) erfolgt ist. Jedoch wird dadurch die Rechnung aufwendiger und die grundsätzlichen Aussagen, die nachfolgend getroffen werden sollen, ändern sich hierdurch nicht, so dass auf eine analytische Darstellung unter Einbeziehung dieser Impedanz-Komponenten verzichtet werden kann.

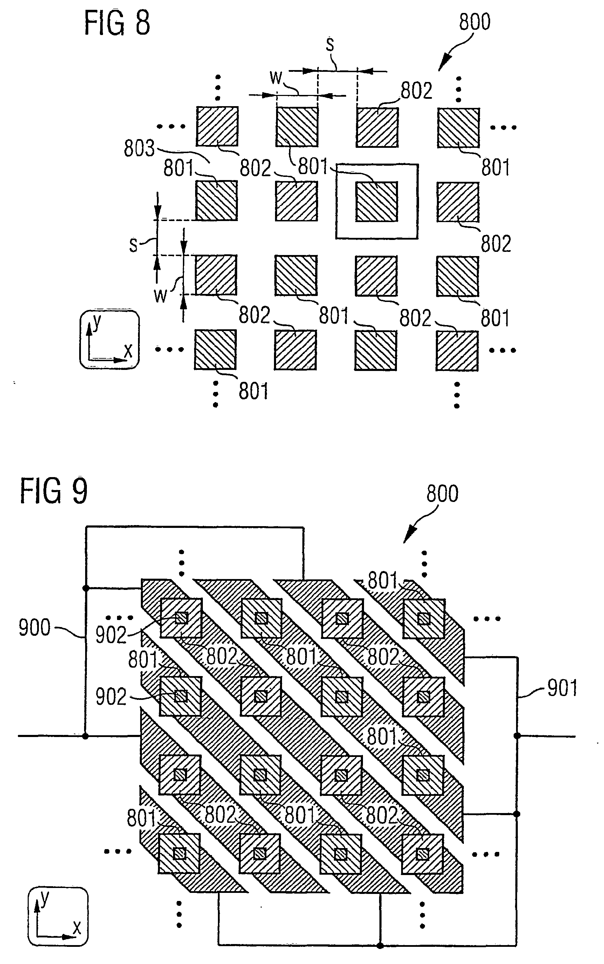

Im Weiteren wird bezugnehmend auf **Fig.8** eine Planar-Sensor-Anordnung 800 gemäß einem ersten Ausführungsbeispiel der Erfindung beschrieben.

Die in Fig.8 gezeigte Planar-Sensor-Anordnung 800 zum Erfassen von in einem Analyten (nicht gezeigt) möglicherweise enthaltenen Partikeln (nicht gezeigt) enthält ein Silizium-Substrat 801. Ferner enthält die Planar-Sensor-Anordnung 800 eine erste planare Sensor-Elektrode und eine zweite planare Sensor-Elektrode, die auf dem Silizium-Substrat 101 gebildet sind, und auf denen Fängermoleküle (nicht gezeigt) immobilisiert sind. Die erste Sensor-Elektrode ist in eine Mehrzahl von planare erste Sensor-Elektroden-Teilbereiche 801 aufgeteilt, und die zweite Sensor-Elektrode ist in eine Mehrzahl von zweite planare Sensor-Elektroden-Teilbereiche 802 aufgeteilt. Die ersten Sensor-Elektroden-Teilbereiche 801 der ersten Sensor-Elektrode und die zweiten Sensor-Elektroden-Teilbereiche 802 der zweiten Sensor-Elektrode sind in der Oberflächenebene des Silizium-Substrat 803 in zwei Dimensionen, nämlich gemäß der Papierebene von Fig.8 entlang Dimension x und entlang Dimension y alternierend angeordnet. Ferner ist eine in Fig.8 nicht gezeigte Verdrahtungsstruktur in dem Substrat vorgesehen (unterhalb der Papierebene von Fig.8), mittels welcher die ersten Sensor-Elektroden-Teilbereiche 801 miteinander gekoppelt sind, und mittels welcher die zweiten Sensor-Elektroden-Teilbereiche 802 miteinander gekoppelt sind.

Es ist anzumerken, dass die Sensor-Elektroden-Teilbereiche 801, 802 planar angeordnet sind, d.h. im Wesentlichen in der Oberflächenebene des Silizium-Substrats 803 gebildet sind und aus dem Silizium-Substrat 803 nicht oder nur ganz geringfügig herausragen. Im Wesentlichen schließt die Oberfläche der planaren Sensor-Elektroden-Teilbereiche 801, 802 mit der Oberfläche des Silizium-Substrats 803 ab.

Anders ausgedrückt wird anstelle einer Anordnung gemäß Fig.1 bis Fig.5 erfindungsgemäß eine Planar-Sensor-Anordnung 800 mit planeren verteilten Elektrodenstrukturen geschaffen. Hierbei werden anstelle von Fingerelektroden 101, 102 Einzelflächen 801, 802 zum Bilden von ersten und zweiten Sensor-Elektroden verwendet, die gemäß Fig.8 in einem schachbrettartigen Muster angeordnet sind. Im Gegensatz zu der Sensor-Anordnung 100 gemäß Fig.1 bis Fig. 5 wird bei der Planar-Sensor-Anordnung 800 eine zusätzliche, unterhalb der Sensor-Ebene angeordnete Verdrahtungsebene bereitgestellt, um die miteinander assoziierten Flächen 801 miteinander elektrisch zu verbinden und um die miteinander assoziierten Flächen 802 miteinander zu verbinden.

Eine solche Verdrahtungsebene ist in **Fig.9** dargestellt, wo nochmals die Planar-Sensor-Anordnung 800 gezeigt ist. Die Verdrahtung gemäß Fig.9 enthält eine erste Verdrahtungsstruktur 900, mittels welcher die ersten planaren Sensor-Elektroden-Teilbereiche 801 miteinander elektrisch gekoppelt sind. Mittels einer zweiten Verdrahtungsstruktur 901 sind die zweiten planaren Sensor-Elektroden-Teilbereiche 802 miteinander gekoppelt. Die erste Verdrahtungsstruktur 900 und eine zweite Verdrahtungsstruktur 901 sind voneinander elektrisch entkoppelt, sind in einer Ebene unterhalb der Ebene der ersten und zweiten planaren Sensor-Elektroden= Teilbereiche 801, 802 realisiert und sind mit den Sensor-Elektroden-Teilbereichen 801, 802 mittels Vias 902, d.h. zu der Papierebene von Fig.9 senkrecht orientierten Kontaktierungselementen, kontaktiert.

Die ersten und zweiten Sensor-Elektroden-Teilbereiche 801, 802 bilden eine matrixförmige Anordnung, wobei die ersten und zweiten Verdrahtungsstrukturen 900, 901 in einem Winkel von 45° zu den Zeilen bzw. Spalten der Matrix verlaufend angeordnet sind. Die Zeilen und die Spalten sind mittels der Sensor-Elektroden-Teilbereiche 801, 802 gebildet.

In Fig.9 stellen die Leiterbahnen, d.h. die schräg verlaufend dargestellten Komponenten, der ersten und zweiten Verdrahtungsstrukturen 900, 901 die Verdrahtung in der unterhalb der Sensor-Ebene liegenden Verdrahtungsebene dar, die Vias 902 bilden die Kontakte zwischen dieser Ebene und den Sensor-Elektroden 801, 802. Da eine Verdrahtungsebene (wie jene, in welcher die ersten und zweiten Verdrahtungsstrukturen 900, 901 gebildet sind) in einem Fertigungsprozess häufig zur Verfügung stehen, insbesondere wenn die Sensor-Anordnung auf Basis eines CMOS-Prozesses gefertigt wird, stellt das Bereitstellen der Verdrahtungsebene unterhalb der Sensor-Elektroden-Teilbereiche 801, 802 keinen oder nur einen sehr geringen Zusatzaufwand dar.

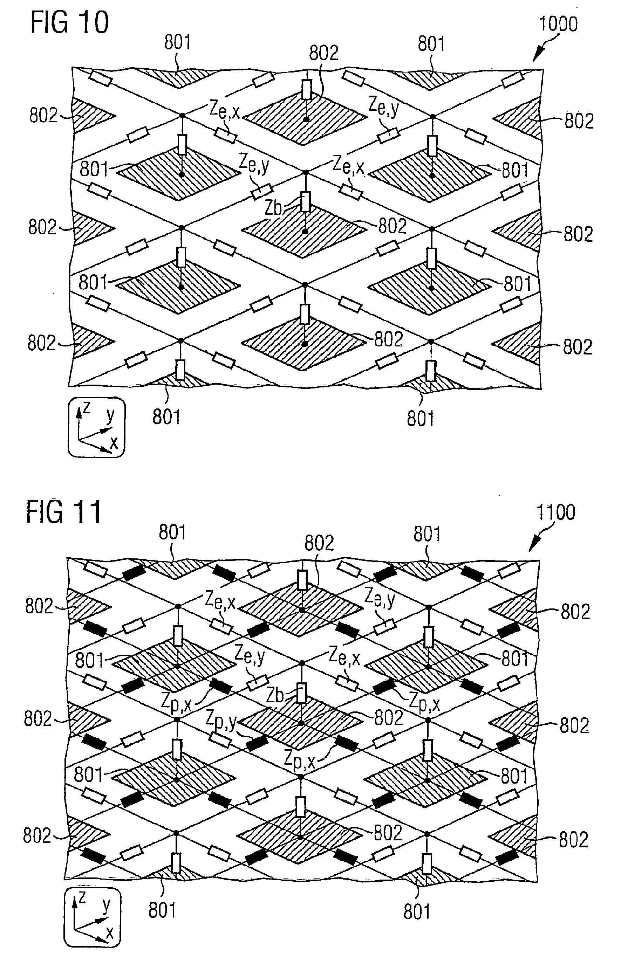

In **Fig.10** ist eine Draufsicht 1000 eines Teilbereichs der Planar-Sensor-Anordnung 800 mit eingezeichnetem Ersatzschaltbild ähnlich wie in Fig.5 gezeigt. Fig.11 zeigt eine andere Draufsicht 1100 mit einer analogen erweiterten Darstellung entsprechend Fig.7. Die eingezeichneten Elemente in Fig.10, Fig.11 haben die entsprechende Bedeutung wie in Fig.5 und Fig.7.

Zur Berechnung der Leistungsfähigkeit der erfindungsgemäßen Planar-Sensor-Anordnung wird im Weiteren eine Berechnung entsprechend Gleichungen (1) bis (19) auf Basis der Betrachtung von Fig.10 durchgeführt, wodurch ein mit einer Elektrode assoziierter Wert der Impedanz erhalten wird:

$$\breve{Z}_{sens} = \breve{Z}_{b,0} \times [(w+s)^2/w^2] + 1/4 Z_{e,sqr} \times [1/2(w+s)/w] \qquad (20)$$

$$Z_{sens} = (1+s/w) \times [Z_{b,0} \times (1+s/w) + 1/8 Z_{e,sqr}] \qquad (21)$$

Für mittlere Frequenzen ergibt sich:

$$Z_{sens}' = (1+s/w) \times [1/j\omega C_{b,0} \times (1+s/w) + 1/8 R_{e,sqr}] \qquad (22)$$

$$|Z_{sens}'| = (1+s/w) \times \sqrt{\left(\frac{1+s/w}{\omega C_{b,0}}\right)^2 + \left(\frac{1}{8} \times R_{e,sqr}\right)^2} \qquad (23)$$

$$Im(Z_{sens}') = 1/\omega C_{b,0} \times (1+s/w)^2 \qquad (24)$$

bzw. für hohe Frequenzen:

$$Z_{sens}'' = 1/j\omega \times (1+s/w) \times [1/C_{b,0} \times (1+s/w) + 1/8 \times 1/C_{e,sqr}] \qquad (25)$$

$$|Z_{sens}''| = 1/\omega \times (1+s/w) \times [1/C_{b,0} \times (1+s/w) + 1/8 \times 1/C_{e,sqr}] \qquad (26)$$

$$Im(Z_{sens}'') = 1/\omega \times (1+s/w) \times [1/C_{b,0} \times (1+s/w) + 1/8 \times 1/C_{e,sqr}] \qquad (27)$$

Um die Sensitivität der Planar-Sensor-Anordnung 800 zu bewerten, werden wieder die relativen Empfindlichkeiten $\eta_{abs}$ bzw. $\eta_{im}$ betrachtet.

Für mittlere Frequenzen wird erhalten:

$$\eta_{abs,2}' = \frac{\partial |z_{sens}|/\partial |z_{b,0}|}{|z_{sens}|} = \frac{\partial \left((1+s/w) \times \sqrt{\left(\frac{1+s/w}{\omega C_{b,0}}\right)^2 + \left(\frac{1}{8} \times R_{e,sqr}\right)^2}\right)\Big/ \partial\left(\frac{1}{\omega C_{b,0}}\right)}{\sqrt{(1+s/w) \times \left(\frac{1+s/w}{\omega C_{b,0}}\right)^2 + \left(\frac{1}{8} \times R_{e,sqr}\right)^2}} \qquad (28)$$

$$\eta_{abs,2}' = \frac{\omega C_{b,0}}{1 + \left(\frac{1}{8} \times \frac{1}{1+s/w} \times \omega C_{b,0} R_{e,sqr}\right)^2} \qquad (29)$$

und

$$\eta_{im,2}' = \frac{\frac{\partial \overline{Im}\,(Z_{sens})}{\partial |Z_{b,0}|}}{\overline{Im}\,(Z_{sens})} = \frac{\partial\left(\frac{(1+s/w)^2}{\omega C_{b,0}}\right) \Big/ \partial\left(\frac{1}{\omega C_{b,0}}\right)}{\left(\frac{(1+s/w)^2}{\omega C_{b,0}}\right)} \tag{30}$$

$$\eta_{im,2}' = \omega C_{b,0} \tag{31}$$

Für hohe Frequenzen wird erhalten:

$$\eta_{abs,2}'' = \frac{\partial |Z_{sens}| / \partial |Z_{b,0}|}{|Z_{sens}|} = \frac{\partial\left[\frac{1}{\omega}\times\left(1/C_{b,0}\times\left(1+\frac{s}{w}\right)+\frac{1}{8}\times 1/C_{e,sqr}\right)\right] \Big/ \partial\left(\frac{1}{\omega\,C_{b,0}}\right)}{\frac{1}{\omega}\times\left(1/C_{b,0}\times\left(1+\frac{s}{w}\right)+\frac{1}{8}\times 1/C_{e,sqr}\right)} \tag{32}$$

$$\eta_{abs,2}'' = \frac{\omega\,C_{b,0}}{1+\frac{1}{8}\times\frac{1}{1+s/w}\times\frac{C_{b,0}}{C_{e,sqr}}} \tag{33}$$

und aufgrund der Identität von Gleichungen (26) und (27)

$$\eta_{im,2}'' = \frac{\frac{\partial Im(Z_{sens})}{\partial |Z_{b,0}|}}{Im(Z_{sens})} = \frac{\omega C_{b,0}}{1+\frac{1}{8}\times\frac{1}{1+s/w}\times\frac{C_{b,0}}{C_{e,sqr}}} \tag{34}$$

Im Weiteren werden die Sensitivitäten der erfindungsgemäßen Planar-Sensor-Anordnung 800 mit verteilten ElektrodenStrukturen und der aus dem Stand der Technik bekannten Konfiguration gemäß Fig. 1 mit Interdigitalelektroden in Beziehung miteinander gesetzt.

Es wird bei der Bewertung der Beträge bei einem Betrieb bei mittleren Frequenzen erhalten:

$$\eta_{abs,2}'/\eta_{abs,1}' = \frac{\omega C_{b,0}}{1+\left(\frac{1}{8}\times\frac{1}{1+s/w}\times\omega C_{b,0}R_{e,sqr}\right)^2} \Big/ \frac{\omega C_{b,0}}{1+\left(\frac{1}{4}\times\frac{1}{1+s/w}\times\omega C_{b,0}R_{e,sqr}\right)^2} \tag{35}$$

$$\eta_{abs,2}{'}/\eta_{abs,1}{'} = \frac{1+4\times\left(\frac{1}{8}\times\frac{1}{1+s/w}\times\omega C_{b,0}R_{e,sqr}\right)^2}{1+\left(\frac{1}{8}\times\frac{1}{1+s/w}\times\omega C_{b,0}R_{e,sqr}\right)^2} \tag{36}$$

$$\eta_{abs,2}{'}/\eta_{abs,1}{'} > 1 \tag{37}$$

Bei Bewertung der Imaginärteile bei einem Betrieb bei mittleren Frequenzen wird erhalten:

$$\eta_{im,2}{'}/\eta_{im,1}{'} = \omega C_{b,0}/\omega C_{b,0} = 1 \tag{38}$$

Die Bewertung der Beträge und des Imaginärteils bei einem Betrieb mit hohen Frequenzen ergibt:

$$\eta_{abs,2}{''}/\eta_{abs,1}{''} = \eta_{im,2}{''}/\eta_{im,1}{''} = \tag{39}$$

$$= \frac{\omega C_{b,0}}{1+\frac{1}{8}\times\frac{1}{1+s/w}\times\frac{C_{b,0}}{C_{e,sqr}}} \Bigg/ \frac{\omega C_{b,0}}{1+\frac{1}{4}\times\frac{1}{1+s/w}\times\frac{C_{b,0}}{C_{e,sqr}}} \tag{40}$$

$$= \frac{1+2\times\left(\frac{1}{8}\times\frac{1}{1+s/w}\times\frac{C_{b,0}}{C_{e,sqr}}\right)}{1+\left(\frac{1}{8}\times\frac{1}{1+s/w}\times\frac{C_{b,0}}{C_{e,sqr}}\right)} \tag{41}$$

$$\eta_{abs,2}{''}/\eta_{abs,1}{''} = \eta_{im,2}{''}/\eta_{im,1}{''} > 1 \tag{42}$$

Aus den Gleichung (37), (38) und (42) ist ersichtlich, dass die Sensor-Konfiguration gemäß der Erfindung den in [4], [7], [8] offenbarten Konfigurationen in allen relevanten Betriebsarten (d.h. insbesondere in allen Frequenzbereichen) gleichwertig bzw. überlegen ist.

Es ist anzumerken, dass die Plausibilitätsrechnung bezugnehmend Gleichungen (1) bis (42) mit einem einfachen Ersatzschaltbild einem Verlauf der Feldlinien im Elektrolyten in der xy-Ebene entspricht, die entweder nur in x-Richtung oder nur in y-Richtung verlaufen.

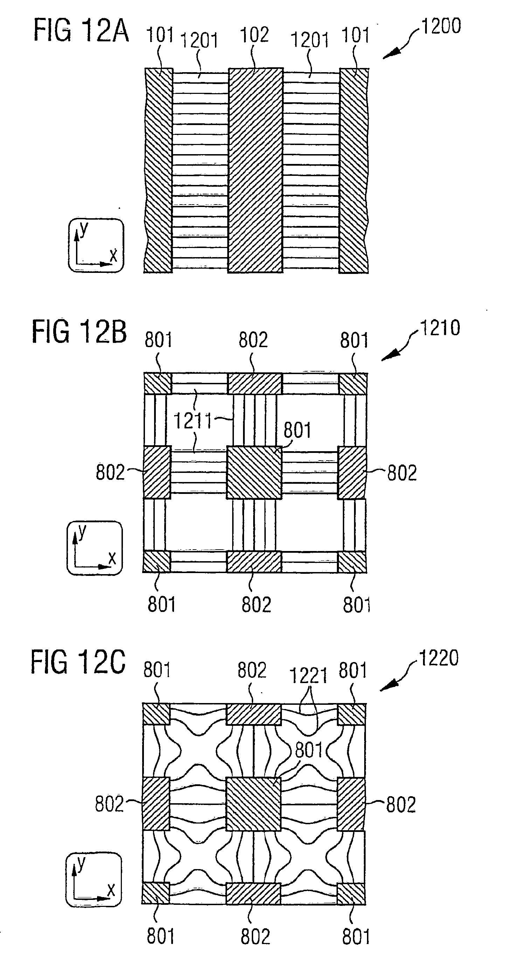

Für die Sensor-Anordnung 100 ist der Feldlinienverlauf 1201 des Teilbereichs 1200 aus **Fig.12A** der Rechnung zugrunde gelegt. Für die erfindungsgemäße Planar-Sensor-Anordnung 800 ist ein Feldlinienverlauf 1211 angenommen, wie er in Teilbereich 1210 in **Fig.12B** gezeigt ist. Die Annahme gemäß Fig.12A ist für die Sensor-Anordnung 100 gemäß Fig.1 bis Fig.5 aus Symmetriegründen sehr realistisch. Für die Planar-Sensor-Anordnung 800 der Erfindung mit verteilten Elektroden gemäß Fig.8 ist jedoch ein Feldlinienverlauf 1221, wie er dem in **Fig.12C** gezeigten Teilbereich 1220 entspricht, realistischer als die idealisierte Darstellung in Fig.12B. Die in Fig.12C gezeigte effektive Verlängerung der Feldlinien 1221 gegenüber der idealisierten rechnerisch einfacheren Darstellung aus Fig.12B führt qualitativ zu einer Vergrößerung der Impedanz des Elektrolyten. Folglich ergeben sich für die Planar-Sensor-Anordnung 800 aus Fig.8 noch größere Werte für den Parameter $\eta$, d.h. der erfindungsgemäße Sensor ist eher noch besser als dies aus Gleichungen (20) bis (42) hervorgeht.

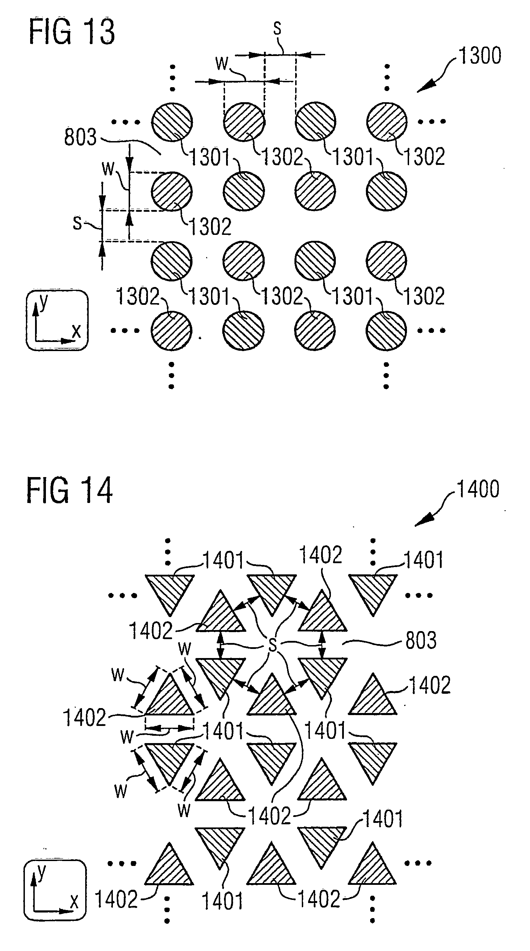

Im Weiteren werden alternative Geometrien für die planaren Sensor-Elektroden-Teilbereiche anhand Fig.13 bis Fig.15 beschrieben. Es ist anzumerken, dass jede beliebige andere Elektrodenkonfiguration möglich ist und die in Fig.8, Fig.13 bis Fig.15 dargestellten Geometrien nur exemplarisch genannt sind.

In **Fig.13** ist eine Planar-Sensor-Anordnung 1300 gemäß einem zweiten Ausführungsbeispiel der Erfindung beschrieben.

Bei der Planar-Sensor-Anordnung 1300 sind die schachbrettartig angeordneten Sensor-Elektroden-Teilbereiche einer ersten Sensor-Elektrode mittels erster planarer runder Sensor-Elektroden-Teilbereiche 1301 und sind die Sensor-Elektroden-Teilbereiche einer zweiten Sensor-Elektrode 1302 mittels zweiter planarer runder Sensor-Elektroden-Teilbereiche 1302 gebildet.

Im Weiteren wird bezugnehmend auf **Fig.14** eine Planar-Sensor-Anordnung 1400 gemäß einem dritten Ausführungsbeispiel der Erfindung beschrieben.

In Fig.14 ist die erste Sensor-Elektrode mittels erster planarer dreieckiger Sensor-Elektroden-Teilbereiche 1401 und ist die zweite Sensor-Elektrode mittels zweiter planarer dreieckiger Sensor-Elektroden-Teilbereiche 1402 gebildet, so dass dreieckförmige Sensor-Elektroden-Teilbereiche vorgesehen sind. Die Sensor-Elektroden-Teilbereiche 1401, 1402 weisen in der Oberflächenebene des Silizium-Substrats 803 die Form von gleichseitigen Dreiecken auf, wobei die Dreieckseiten benachbarter Sensor-Elektroden-Teilbereiche zueinander parallel angeordnet sind.

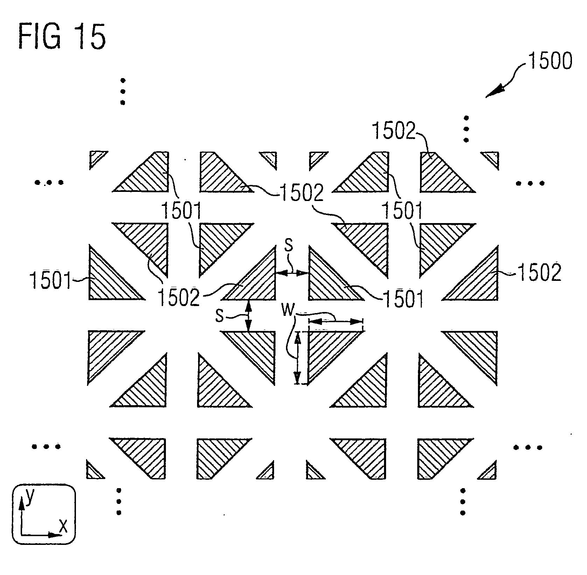

Im Weiteren wird bezugnehmend auf **Fig.15** eine Planar-Sensor-Anordnung 1500 gemäß einem vierten Ausführungsbeispiel der Erfindung beschrieben.

Bei der Planar-Sensor-Anordnung 1500 sind die ersten und zweiten Sensor-Elektroden wiederum mittels erster planarer dreieckiger Sensor-Elektroden-Teilbereiche 1501 und mittels zweiter planarer dreieckiger Sensor-Elektroden-Teilbereiche 1502 realisiert. Die Sensor-Elektroden-Teilbereiche 1501, 1502 in der Oberflächenebene des Silizium-Substrats 803 weisen die Gestalt von rechtwinkligen Dreiecken auf, wobei die Dreieckseiten benachbarter Sensor-Elektroden-Teilbereiche 1501, 1502 zueinander parallel angeordnet sind.

In diesem Dokument sind folgende Veröffentlichungen zitiert:

[1] Paeschke, M et al. (1996), Electroanalysis 1996(7), Nr.1, S.1-8

[2] Hintzsche, R et al. (1997) "Microbiosensors using electrodes made in Si-technology", In: "Frontiers in Biosensorics I - Fundamental Aspects", Scheller, FW et al. (eds.), Birkhauser Verlag Basel

[3] WO 93/22678

[4] DE 196 10 115 A1

[5] Van Gerwen, P et al. (1997) "Transducers" '97", S.907-910

[6] Krause, C et al. (1996), Langmuir, Band 12, Nr. 25, S.6059-6064

[7] WO 01/43870 A2

[8] WO 97/21094

[9] Thewes, R et al. (April 2004) "CMOS Sensor Interface Arrays for DNA Detection", Proc 13th Workshop on Advances in Analog Circuit Design (AACD)

[10] WO 03/083134

[11] WO 2004/019024 bzw. EP 1445609

[12] WO 01/75151 A2

[13] WO 03/102602 A2

[14] US 6,383,858 B1

Bezugszeichenliste

| | |
|---|---|
| 100 | Sensor-Anordnung |
| 101 | erste Interdigitalelektrode |
| 102 | zweite Interdigitalelektrode |
| 103 | Silizium-Substrat |
| 110 | Querschnitts-Ansicht |
| 111 | Teilbereich |
| 200 | Fängermoleküle |
| 201 | Impedanz |
| 202 | zu erfassende Partikel |
| 300 | Teilansicht |
| 301 | Feldlinienverlauf |
| 302 | Symmetrielinien |
| 400 | Teilbereich |
| 500 | Draufsicht |
| 600 | Ersatzschaltbild |
| 700 | Draufsicht |
| 800 | Planar-Sensor-Anordnung |
| 801 | erste planare Sensor-Elektroden-Teilbereiche |
| 802 | zweite planare Sensor-Elektroden-Teilbereiche |
| 803 | Silizium-Substrat |
| 900 | erste Verdrahtungsstruktur |
| 901 | zweite Verdrahtungsstruktur |
| 902 | Vias |
| 1000 | Draufsicht |
| 1100 | Draufsicht |
| 1200 | Teilbereich |
| 1201 | Feldlinienverlauf |
| 1210 | Teilbereich |
| 1211 | Feldlinienverlauf |

1220    Teilbereich

1221    Feldlinienverlauf

1300    Planar-Sensor-Anordnung

1301    erste planare runde Sensor-Elektroden-Teilbereiche

1302    zweite planare runde Sensor-Elektroden-Teilbereiche

1400    Planar-Sensor-Anordnung

1401    erste planare dreieckige Sensor-Elektroden-Teilbereiche

1402    zweite planare dreieckige Sensor-Elektroden-Teilbereiche

1500    Planar-Sensor-Anordnung

1501    erste planare dreieckige Sensor-Elektroden-Teilbereiche

1502    zweite planare dreieckige Sensor-Elektroden-Teilbereiche


**Patentansprüche**

1.  Planar-Sensor-Anordnung (800) zum Erfassen von in einem Analyten möglicherweise enthaltenen Partikeln

    • mit einem Substrat (803);
    • mit einer ersten planaren Sensor-Elektrode (801) und mit einer zweiten planaren Sensor-Elektrode (802), die auf und/oder in dem Substrat (803) gebildet sind, und auf denen Fängermoleküle immobilisiert sind, wobei die erste Sensor-Elektrode (801) und die zweite Sensor-Elektrode (802) jeweils in eine Mehrzahl von planaren Sensor-Elektroden-Teilbereichen (801, 802) aufgeteilt sind, wobei die Sensor-Elektroden-Teilbereiche (801) der ersten Sensor-Elektrode (801) und die Sensor-Elektroden-Teilbereiche (802) der zweiten Sensor-Elektrode (802) in der Oberflächenebene des Substrats (803) in zwei Dimensionen alternierend angeordnet sind;
    • mit einer Verdrahtungsstruktur (900, 901), mittels welcher zumindest ein Teil der Sensor-Elektroden-Teilbereiche (801) der ersten Sensor-Elektrode (801) miteinander elektrisch gekoppelt sind, und mittels welcher zumindest ein Teil der Sensor-Elektroden-Teilbereiche (802) der zweiten Sensor-Elektrode (802) miteinander elektrisch gekoppelt sind.

2.  Planar-Sensor-Anordnung (800) nach Anspruch 1,
    bei der die Sensor-Elektroden-Teilbereiche (801, 802) im Wesentlichen matrixförmig angeordnet sind.

3.  Planar-Sensor-Anordnung (800) nach Anspruch 1 oder 2,
    bei der zumindest ein Teil der Sensor-Elektroden-Teilbereiche (801, 802) in der Oberflächenebene des Substrats eine Ausdehnung aufweist, die kleiner ist als der Abstand zu mindestens einem unmittelbar benachbarten Sensor-Elektroden-Teilbereich (801, 802).

4.  Planar-Sensor-Anordnung (800) nach einem der Ansprüche 1 bis 3,
    bei der auf der ersten planaren Sensor-Elektrode (801) und/oder auf der zweiten planaren Sensor-Elektrode (802) Fängermoleküle immobilisiert sind.

5.  Planar-Sensor-Anordnung (800) nach einem der Ansprüche 1 bis 4,
    bei der zumindest ein Teil der Sensor-Elektroden-Teilbereiche (801, 802) in der Oberflächenebene des Substrats (803) polygone Gestalt aufweist.

6.  Planar-Sensor-Anordnung (800) nach Anspruch 5,
    bei der zumindest ein Teil der Sensor-Elektroden-Teilbereiche (801, 802)in der Oberflächenebene des Substrats (803) rechteckige oder quadratische Gestalt aufweist.

**7.** Planar-Sensor-Anordnung (1400) nach Anspruch 5 oder 6, bei der zumindest ein Teil der Sensor-Elektroden-Teilbereiche (1401, 1402) in der Oberflächenebene des Substrats (803) dreieckige Gestalt aufweist.

**8.** Planar-Sensor-Anordnung (1400) nach Anspruch 7,
bei der zumindest ein Teil der Sensor-Elektroden-Teilbereiche (1401, 1402) in der Oberflächenebene des Substrats (803) die Gestalt von gleichseitigen Dreiecken aufweist, wobei die Dreiecksseiten benachbarter Sensor-Elektroden-Teilbereiche (1401, 1402) zueinander parallel angeordnet sind.

**9.** Planar-Sensor-Anordnung (1500) nach Anspruch 7,
bei der zumindest ein Teil der Sensor-Elektroden-Teilbereiche (1501, 1502) in der Oberflächenebene des Substrats die Gestalt von rechtwinkligen Dreiecken aufweist, wobei die Dreiecksseiten benachbarter Sensor-Elektroden-Teilbereiche (1501, 1502) zueinander parallel angeordnet sind.

**10.** Planar-Sensor-Anordnung (1300) nach einem der Ansprüche 1 bis 4,
bei der zumindest ein Teil der Sensor-Elektroden-Teilbereiche (1301, 1302) in der Oberflächenebene des Substrats (803) runde oder elliptische Gestalt aufweist.

**11.** Planar-Sensor-Anordnung (800) nach einem der Ansprüche 1 bis 10,
bei der die Verdrahtungsstruktur (900, 901) als vergrabene elektrisch leitfähige Struktur im Inneren des Substrats (803) gebildet ist.

**12.** Planar-Sensor-Anordnung (800) nach Anspruch 11,
bei der in der Ebene, in der die Verdrahtungsstruktur (900, 901) als vergrabene elektrisch leitfähige Struktur gebildet ist, mindestens ein zusätzliches integriertes Bauelement gebildet ist.

**13.** Planar-Sensor-Anordnung (800) nach Anspruch 12,
bei der das mindestens eine zusätzliche integrierte Bauelement ein CMOS-Bauelement ist.

**14.** Planar-Sensor-Anordnung (800) nach einem der Ansprüche 1 bis 13,
bei der die Sensor-Elektroden-Teilbereiche (801, 802) der ersten Sensor-Elektrode (801) und der zweiten Sensor-Elektrode (802) in zwei Dimensionen alternierend und matrixförmig in Zeilen und Spalten angeordnet sind, und bei der die Verdrahtungsstruktur (900, 901) entlang einer Verlaufsrichtung gebildet ist, die mit Verlaufsrichtungen der Zeilen und Spalten einen Winkel von im Wesentlichen 45° einschließt.

**15.** Planar-Sensor-Anordnung (800) nach einem der Ansprüche 1 bis 14,
eingerichtet als Biosensor-Anordnung.

**16.** Planar-Sensor-Anordnung (800) nach einem der Ansprüche 1 bis 15,
eingerichtet als Impedanz-Sensor-Anordnung.

**17.** Planar-Sensor-Anordnung (800) nach einem der Ansprüche 1 bis 16,
eingerichtet als monolithisch integrierte Sensor-Anordnung.

**18.** Planar-Sensor-Anordnung (800) nach einem der Ansprüche 1 bis 17,
mit einer Schaltkreis-Einrichtung, mittels welcher die erste Sensor-Elektrode (801) und die zweite Sensor-Elektrode (802) ansteuerbar ist.

**19.** Planar-Sensor-Anordnung (800) nach Anspruch 18,
bei der mittels der Schaltkreis-Einrichtung an die Sensor-Elektroden (801, 802) ein zeitlich veränderliches elektrisches Ansteuersignal anlegbar ist und von den Sensor-Elektroden (801, 802) ein zeitlich veränderliches elektrisches Detektionssignal erfassbar ist.

**20.** Planar-Sensor-Anordnung (800) nach einem der Ansprüche 1 bis 19,
mit mindestens einem auf und/oder in dem Substrat (803) gebildeten Verstärker-Element und/oder mit mindestens einem auf und/oder in dem Substrat gebildeten Auswerte-Element.

**21.** Sensor-Array,
mit einer Mehrzahl von auf und/oder in dem Substrat gebildeten Planar-Sensor-Anordnungen (800) nach einem der Ansprüche 1 bis 20.

**22.** Verfahren zum Herstellen einer Planar-Sensor-Anordnung (800) zum Erfassen von in einem Analyten möglicherweise enthaltenen Partikeln,
bei dem

• eine erste planare Sensor-Elektrode (801) und eine zweite planare Sensor-Elektrode (802) auf und/oder in einem Substrat (803) gebildet werden, auf denen Fängermoleküle immobilisiert werden, wobei die erste Sensor-Elektrode (801) und die zweite Sensor-Elektrode (802) jeweils in eine Mehrzahl von planaren Sensor-Elektroden-Teilbereichen (801, 802) aufgeteilt werden, wobei die Sensor-Elektroden-Teilbereiche (801) der ersten Sensor-Elektrode (801) und die Sensor-Elektroden-Teilbereiche (802) der zweiten Sensor-Elektrode (802) in der Oberflächenebene des Substrats (803) in zwei Dimensionen alternierend angeordnet werden;
• eine Verdrahtungsstruktur (900) gebildet wird, mittels welcher zumindest ein Teil der Sensor-Elektroden-Teilbereiche (801) der ersten Sensor-Elektrode (801) miteinander elektrisch gekoppelt werden, und mittels welcher zumindest ein Teil der Sensor-Elektroden-Teilbereiche (802) der zweiten Sensor-Elektrode (802) miteinander elektrisch gekoppelt werden.

**Claims**

**1.** Planar sensor arrangement (800) for detecting particles possibly contained in an analyte,

• comprising a substrate (803);
• comprising a first planar sensor electrode (801) and comprising a second planar sensor electrode (802) which are formed on and/or in the substrate (803) and on which catcher molecules are immobilized, the first sensor electrode (801) and the second sensor electrode (802) in each case being divided into a plurality of planar sensor electrode partial regions (801, 802), the sensor electrode partial regions (801) of the first sensor electrode (801) and the sensor electrode partial regions (802) of the second sensor electrode (802) being arranged alternately in two dimensions in the surface plane of the substrate (803);
• comprising a wiring structure (900, 901) by means of which at least one portion of the sensor electrode partial regions (801) of the first sensor electrode (801) are electrically coupled to one another and by means of which at least one portion of the sensor electrode partial regions (802) of the second sensor electrode (802) are electrically coupled to one another.

**2.** Planar sensor arrangement (800) according to Claim 1,
in which the sensor electrode partial regions (801, 802) are arranged essentially in matrix-type fashion.

**3.** Planar sensor arrangement (800) according to Claim 1 or 2, in which at least one portion of the sensor electrode partial regions (801, 802) in the surface plane of the subrate has an extent which is less than the distance from at least one directly adjacent sensor electrode partial region (801, 802).

**4.** Planar sensor arrangement (800) according to one of Claims 1 to 3,
in which catcher molecules are immobilized on the first planar sensor electrode (801) and/or on the second planar sensor electrode (802).

**5.** Planar sensor arrangement (800) according to one of Claims 1 to 4,
in which at least one portion of the sensor electrode partial regions (801, 802) in the surface plane of the substrate (803) has polygonal shape.

**6.** Planar sensor arrangement (800) according to Claim 5,
in which at least one portion of the sensor electrode partial regions (801, 802) in the surface plane of the substrate (803) has rectangular or square shape.

**7.** Planar sensor arrangement (1400) according to Claim 5 or 6,
in which at least one portion of the sensor electrode partial regions (1401, 1402) in the surface plane of the substrate (803) has triangular shape.

**8.** Planar sensor arrangement (1400) according to Claim 7,

in which at least one portion of the sensor electrode partial regions (1401, 1402) in the surface plane of the substrate (803) has the shape of equilateral triangles, the triangle sides of adjacent sensor electrode partial regions (1401, 1402) being arranged parallel to one another.

9. Planar sensor arrangement (1500) according to Claim 7,
in which at least one portion of the sensor electrode partial regions (1501, 1502) in the surface plane of the substrate has the shape of right-angled triangles, the triangle sides of adjacent sensor electrode partial regions (1501, 1502) being arranged parallel to one another.

10. Planar sensor arrangement (1300) according to one of Claims 1 to 4,
in which at least one portion of the sensor electrode partial regions (1301, 1302) in the surface plane of the subrate (803) has round or elliptical shape.

11. Planar sensor arrangement (800) according to one of Claims 1 to 10,
in which the wiring structure (900, 901) is formed as a buried electrically conductive structure within the substrate (803).

12. Planar sensor arrangement (800) according to Claim 11,
in which at least one additional integrated component is formed in the plane in which the wiring structure (900, 901) is formed as buried electrically conductive structure.

13. Planar sensor arrangement (800) according to Claim 12,
in which the at least one additional integrated component is a CMOS component.

14. Planar sensor arrangement (800) according to one of Claims 1 to 13,
in which the sensor electrode partial regions (801, 802) of the first sensor electrode (801) and of the second sensor electrode (802) are arranged alternately in two dimensions and in matrix-type fashion in rows and columns, and in which the wiring structure (900, 901) is formed along a course direction which forms an angle of essentially 45° with course directions of the rows and columns.

15. Planar sensor arrangement (800) according to one of Claims 1 to 14,
set up as a biosensor arrangement.

16. Planar sensor arrangement (800) according to one of Claims 1 to 15,
set up as an impedance sensor arrangement.

17. Planar sensor arrangement (800) according to one of Claims 1 to 16,
set up as a monolithically integrated sensor arrangement.

18. Planar sensor arrangement (800) according to one of Claims 1 to 17,
comprising a circuit device by means of which the first sensor electrode (801) and the second sensor electrode (802) can be driven.

19. Planar sensor arrangement (800) according to Claim 18,
in which, by means of the circuit device, a temporally variable electrical drive signal can be applied to the sensor electrodes (801, 802) and a temporally variable electrically detection signal can be detected by the sensor electrodes (801, 802).

20. Planar sensor arrangement (800) according to one of Claims 1 to 19,
comprising at least one amplifier element formed on and/or in the substrate (803) and/or comprising at least one evaluation element formed on and/or in the substrate.

21. Sensor array
comprising a plurality of planar sensor arrangements (800) according to one of Claims 1 to 20 which are formed on and/or in the substrate.

22. Method for the production of a planar sensor arrangement (800) for detecting particles possibly contained in an analyte, in which

• a first planar sensor electrode (801) and a second planar sensor electrode (802) are formed on and/or in a substrate (803), on which electrodes catcher molecules are immobilized, the first sensor electrode (801) and the second sensor electrode (802) in each case being divided into a plurality of planar sensor electrode partial regions (801, 802), the sensor electrode partial regions (801) of the first sensor electrode (801) and the sensor electrode partial regions (802) of the second sensor electrode (802) being arranged alternately in two dimensions in the surface plane of the substrate (803);

• a wiring structure (900) is formed by means of which at least one portion of the sensor electrode partial regions (801) of the first sensor electrode (801) are electrically coupled to one another and by means of which at least one portion of the sensor electrode partial regions (802) of the second sensor electrode (802) are electrically coupled to one another.

## Revendications

1. Agencement ( 800 ) détecteur plan pour la détection de particules contenues éventuellement dans un analyte

   • comprenant un substrat ( 803 ) ;
   • comprenant une première électrode (801) de détecteur plane et comprenant une deuxième électrode (802) de détecteur plane, qui sont formées sur et/ou dans le substrat ( 803 ) et sur lesquelles sont immobilisées des molécules de capture, la première électrode (801) de détecteur et la deuxième électrode ( 802 ) de détecteur étant subdivisées respectivement en une pluralité de zones ( 801, 802 partielles planes d'électrodes de détecteur, les zones ( 801 ) partielles d'électrodes de détecteur de la première électrode ( 801 ) de détecteur et les zones ( 802 ) partielles d'électrodes de détecteur de la deuxième électrode ( 802 ) de détecteur étant disposées en alternance en deux dimensions dans le plan de la surface du substrat ( 803 ) ;
   • comprenant une structure (900, 901) de câblage, au moyen de laquelle au moins une partie des zones ( 801 ) partielles d'électrodes de détecteur de la première électrode ( 801 ) de détecteur sont reliées électriquement entre elles et au moyen de laquelle au moins une partie des zones (802) partielles d'électrodes de détecteur de la deuxième électrode (802) de détecteur sont reliées électriquement entre elles.

2. Agencement (800) détecteur plan suivant la revendication 1,
   dans lequel les zones ( 801, 802 ) partielles d'électrodes de détecteurs sont disposées sensiblement sous la forme d'une matrice.

3. Agencement (800) détecteur plan suivant la revendication 1 ou 2,
   dans lequel au moins une partie des zones (801, 802) partielles d'électrodes de détecteur ont dans le plan de la surface du substrat une étendue qui est plus petite que la distance à au moins une zone (801, 802) partielle directement voisine d'électrodes de détecteur.

4. Agencement (800) détecteur plan suivant l'une des revendications 1 à 3,
   dans lequel des molécules de capture sont immobilisées sur la première électrode (801) de détecteur plane et/ou sur la deuxième électrode ( 802 ) de détecteur plane.

5. Agencement (800) détecteur plan suivant l'une des revendications 1 à 4,
   dans lequel au moins une partie des zones (801, 802) partielles d'électrodes de détecteur a une forme polygonale dans le plan de la surface du substrat ( 803 ).

6. Agencement (800) détecteur plan suivant la revendication 5,
   dans lequel au moins une partie des zones (801, 802) partielles d'électrodes de détecteur a une forme rectangulaire ou carrée dans le plan de la surface du substrat ( 803 ).

7. Agencement (1400) détecteur plan suivant la revendication 5 ou 6,
   dans lequel au moins une partie des zones (801, 802 ) partielles d'électrodes de détecteur a une forme triangulaire dans le plan de la surface du substrat ( 803 ).

8. Agencement (1400) détecteur plan suivant la revendication 7,
   dans lequel au moins une partie des zones (1401, 1402) partielles d'électrodes de détecteur a la forme de triangle équilatéral dans le plan de la surface du substrat ( 803 ), les côtés de triangle de zones ( 1401, 1402 ) partielles d'électrodes de détecteur voisines étant disposés parallèlement les uns aux autres.

**9.** Agencement (1500) détecteur plan suivant la revendication 7,
dans lequel au moins une partie des zones (1501, 1502) partielles d'électrodes de détecteur a la forme de triangle rectangle dans le plan de la surface du substrat, les côtés de triangle de zones ( 1501, 1502 ) partielles d'électrodes de détecteur voisines étant disposés parallèlement entre eux.

**10.** Agencement (1300) détecteur plan suivant l'une des revendications 1 à 4,
dans lequel au moins une partie des zones (1301, 1302) partielles d'électrodes de détecteur a une forme circulaire ou elliptique dans le plan de la surface du substrat ( 803 ).

**11.** Agencement (800) détecteur plan suivant l'une des revendications 1 à 10,
dans lequel la structure ( 900, 901 ) de câblage est sous la forme d'une structure conductrice de l'électricité enterrée à l'intérieur du substrat ( 803 ).

**12.** Agencement (800) détecteur plan suivant la revendication 11,
dans lequel au moins un composant intégré supplémentaire est formé dans le plan dans lequel la structure ( 900, 901 ) de câblage est sous la forme d'une structure enterrée conductrice de l'électricité.

**13.** Agencement (800) détecteur plan suivant la revendication 12,
dans lequel le au moins un composant intégré supplémentaire est un composant CMOS.

**14.** Agencement (800) détecteur plan suivant l'une des revendications 1 à 13,
dans lequel les zones ( 801, 802 ) partielles d'électrodes de détecteur de la première électrode ( 801 ) de détecteur et de la deuxième électrode ( 802 ) de détecteur sont disposées en alternance suivant deux dimensions et sous forme de matrice en lignes et en colonnes et dans lequel la structure ( 900, 901 ) de câblage s'étend suivant une direction, qui fait un angle de sensiblement 45° avec les directions dans lesquelles s'étendent les lignes et les colonnes.

**15.** Agencement (800) détecteur plan suivant l'une des revendications 1 à 14,
agencé en agencement de biodétecteur.

**16.** Agencement (800) détecteur plan suivant l'une des revendications 1 à 15,
agencé en agencement de détecteur d'impédance.

**17.** Agencement (800) détecteur plan suivant l'une des revendications 1 à 16,
agencé en agencement de détecteur intégré monolithiquement.

**18.** Agencement (800) détecteur plan suivant l'une des revendications 1 à 17,
comprenant un dispositif de circuit, au moyen duquel la première électrode (801) de détecteur et la deuxième électrode ( 802 ) de détecteur peuvent être commandées.

**19.** Agencement (800) détecteur plan suivant la revendication 18,
dans lequel au moyen du dispositif de circuit un signal électrique de commande, qui peut être modifié dans le temps, peut être appliqué aux électrodes ( 801, 802 ) de détecteur et un signal électrique de détection, qui peut être modifié dans le temps, peut être détecté par les électrodes ( 801, 802 ) de détecteur.

**20.** Agencement (800) détecteur plan suivant l'une des revendications 1 à 19,
comprenant au moins un élément amplificateur formé sur et/ou dans le substrat ( 803 ) et/ou comprenant au moins un élément d'exploitation formé sur et/ou dans le substrat.

**21.** Réseau détecteur,
comprenant une pluralité d'agencements ( 800 ) détecteur plan formés dans le substrat suivant l'une des revendications 1 à 20.

**22.** Procédé de fabrication d'un agencement ( 800 ) détecteur plan pour la détection de particules contenues éventuellement dans un analyte,
dans lequel

  • on forme une première électrode (801) de détecteur plane et une deuxième électrode (802) de détecteur plane sur et/ou dans un substrat ( 803 ), sur lesquelles des molécules de capture sont immobilisées, la première électrode ( 801 ) de détecteur et la deuxième électrode ( 802 ) de détecteur étant subdivisées respectivement en une pluralité de zones ( 801, 802) partielles planes d'électrodes de détecteur, les zones ( 801 ) partielles d'électrodes de détecteur de la première électrode ( 801) de détecteur et les zones ( 802 ) partielles d'électrodes de détecteur de la deuxième électrode ( 802 ) de détecteur étant disposées en alternance suivant deux dimensions dans le plan de la surface du substrat ( 803 ) ;

• on forme une structure ( 900 ) de câblage, au moyen de laquelle au moins une partie des zones ( 801 ) partielles d'électrodes de détecteur de la première électrode (801) de détecteur sont reliées électriquement entre elles et au moyen de laquelle au moins une partie des zones ( 802 ) partielles d'électrodes de détecteur de la deuxième électrode (802) de détecteur sont reliées électriquement entre elles.

FIG 1A   Stand der Technik

FIG 1B   Stand der Technik

FIG 2A  Stand der Technik

FIG 2B  Stand der Technik

FIG 3  Stand der Technik

FIG 4

FIG 5

FIG 6

FIG 7

FIG 8

FIG 9

## FIG 10

## FIG 11

FIG 12A

101 1201 102 1201 101 1200

y
x

FIG 12B

801 802 801 1210

1211 801

802 802

y
x

801 802 801

FIG 12C

801 802 1221 801 1220

801

802 802

y
x

801 802 801

FIG 13

FIG 14

FIG 15

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9322678 A
- DE 19610115 A1
- WO 0143870 A2
- WO 9721094 A
- WO 03083134 A
- WO 2004019024 A
- EP 1445609 A
- WO 0175151 A2
- WO 03102602 A2
- US 6383858 B1

**In der Beschreibung aufgeführte Nicht-Patentliteratur**

- **PAESCHKE, M et al.** *Electroanalysis,* 1996, 1-8
- Microbiosensors using electrodes made in Si-technology. **HINTZSCHE, R et al.** Frontiers in Biosensorics I - Fundamental Aspects. Birkhauser Verlag, 1997
- **VAN GERWEN, P et al.** *Transducers,* 1997, 907-910
- **KRAUSE, C et al.** *Langmuir,* 1996, vol. 12 (25), 6059-6064
- **THEWES, R et al.** CMOS Sensor Interface Arrays for DNA Detection. *Proc 13th Workshop on Advances in Analog Circuit Design (AACD,* April 2004